# EUROPEAN PATENT APPLICATION

(11) **EP 4 095 144 A1**
(43) Date of publication of application: **30.11.2022**
(21) Application number: 21745151.7
(22) Date of filing: 22.01.2021
(51) Int. Cl.: C07F 7/28, C07F 15/02, C07C 63/307, B01J 21/06

(54) **TITANIUM-IRON MOF SOLID, PROCESS FOR OBTAINING IT AND USE THEREOF FOR THE DEGRADATION OF COMPOUNDS**

(30) Priority: 22.01.2020 ES 202030047
(71) Applicant: Universitat de Valéncia, 46010 Valencia (ES); Universidad de Granada, 18010 Granada (ES)
(72) Inventor: MARTÍ GASTALDO, Carlos, 46010 Valencia (ES); CASTELLS GIL, Javier, 46010 Valencia (ES); NEYVIS ALMORA, Barriós, Valencia 46010 (CU); TATAY AGUILAR, Sergio, 46010 Valencia (ES); MUÑOZ PADIAL, Natalia, 18010 Granada (ES); GIL SAN MILLÁN, Rodrigo, 18010 Granada (ES); RODRÍGUEZ NAVARRO, Jorge Andrés, 18010 Granada (ES)
(74) Representative: Ponti & Partners, S.L.P
(86) International application number: PCT/ES2021/070041
(87) International publication number: WO 2021/148702

(57) **Abstract**

The titanium (IV) and iron (III) MOF solid MUV-17 (TiFe₂), has general formula (1): [Ti^{IV}Fe^{III}₂(O)(L)₂(X)₃]S, where X is each equal or different selected from: O²⁻, OH⁻, H₂O, F⁻, Cl⁻, Br, I⁻, NO₃⁻, ClO₄⁻, BF₄⁻, SCN⁻, OH⁻, CH₃COO⁻, C₅H₇O₂⁻, SO₄²⁻ and CO₃²⁻, L is a tricarboxylic ligand and S is at least one molecule of a polar solvent selected from the group consisting of *N,N'*-dimethylformamide*,N,N'* -diethylformamide*,N,N'*-dimethylacetamide, *N*- methyl-2-pyrrolidone, methanol, ethanol, isopropanol, n-propanol, water and mixtures thereof. The titanium (IV) and iron (III) MOF solid has long-term catalytic activity for the degradation of toxic compounds. The method for obtaining them comprises dissolving the components under anaerobic conditions. The invention also relates to the use of the titanium (IV) and iron (III) MOF solid as an additive with detoxifying properties of toxic compounds.

## Description

The present invention belongs to the field of Metal-Organic Frameworks (MOFs).

In particular, the invention relates to a MOF solid, which includes in the metal-organic network titanium (IV) and iron (III), with catalytic activity for the degradation of toxic compounds, as well as a process for obtaining it. The invention also relates to the use of the MOF solid of the invention as an additive with detoxification properties for toxic compounds.

### Background of the invention

Compounds with toxic effects on humans are used increasingly in industry, chemical warfare or agriculture; for this reason, methods and/or products that degrade these compounds have been investigated to protect humans from these toxic compounds since decades ago.

The process by which toxic substances are removed from the body is generally known as detoxification. Detoxification occurs naturally in the body, which neutralizes and eliminates toxins. Fat-soluble toxic compounds such as drugs, insecticides, herbicides, medicaments, industrial compounds, etc., are mainly detoxified in the liver, although such detoxification may also occur in the intestine, kidneys, skin, and lungs.

Among the toxic compounds are organophosphorus compounds that are commonly used as insecticides and as a chemical or nerve agents in war. When used as a chemical agent, the main problem for humans is exposure without effective protection.

The high toxicity of organophosphorus compounds and their use as a common insecticide generates problems of cross-contamination of food, as well as problems of toxicity in humans exposed to these compounds or to materials in contact with these compounds, which makes it essential to completely eliminate these residues or unused material. Nerve agents based on phosphonate groups are potential inhibitors of the acetylcholinesterase (AChE) enzyme of the central nervous system, which causes continuous stimulation of nerve fibers and can result in asphyxiation or even death. Generally, these molecules are characterized by having a P(V) stereogenic center consisting of a P=O double bond, an alkyl (-R) and/or halogen (-X) group, and two alkoxy (-OR) groups. These compounds are degraded by replacing one of their P-X or P-OR bonds with a P-OH bond that results in the formation of the corresponding non-toxic phosphates or alkylphosphonic acids.

Currently, the most widely used product as a filter or protection against organophosphorus compounds is based on activated carbon. These materials are adsorbents but do not degrade the compound, so the material used as a filter or protection cannot be regenerated, is saturated and must be discarded, as is the case, for example, with laboratory masks that after use are impregnated with undegraded toxic compound, becoming a toxic residue.

Materials for protection have been investigated such as, for example, MOF [Ni₈(OH)₄(H₂O)₂(L5-CF3)₆] with improved properties with respect to activated carbon. However, this MOF is intended for the capture of toxic organic compounds and not for their degradation or conversion to non-toxic compounds.

Although MOFs capable of degrading organophosphorus compounds have been described, these require the presence of a buffered basic medium and, in some cases, also the activation of the MOF prior to its use. In addition, there are certain associated technical difficulties such as the complexity of manufacturing materials containing such MOFs in, for example, protective filters, or the need for material regeneration since the catalytic activity of the MOF depends on the presence of a dissolving co-catalyst. These requirements compromise both the degradation efficiency of the catalyst and its regeneration and reusability (or cyclability), which results in a loss of activity and/or reduction of its useful life, greatly limiting its use as protective materials.

As is known, MOFs are a class of porous solids made up of metal atoms or clusters of metal atoms coordinated with organic ligands that create open crystal structures with permanent porosity. Given the wide variety of metal atoms and organic ligands, there is a constant interest in the search for new metal-organic structures with properties designed for specific uses such as catalysis, separation and storage of gases or water treatment and purification.

The most widely used MOFs for the decomposition of toxic agents used in chemical warfare are based, for the most part, on Zr(IV) such as NU-1000, MOF-808 and UiO-66 and other MOFs with metallic centers with vacant positions such as MIL-101 (Cr) or HKUST-1. In this regard, Coordination Chemistry Reviews 2017, 346, 101-111*.* Metal-Organic Frameworks for the Removal of Toxic Industrial Chemicals and Chemical Warfare Agents. Chemical Society Reviews 2017, 46 (11), 3357-3385 discloses the use of MOFs of Zr(IV) for the treatment of chemical warfare agents and their analogues.

Despite the existence of MOFs capable of activating organophosphorus molecules for subsequent degradation, these MOFs are only capable of hydrolyzing/degrading toxic compounds in the presence of a basic buffer solution which is responsible for regenerating the Zr(IV) MOF catalyst. When the reaction is carried out in the absence of the basic medium, for example, in an aqueous medium, the catalytic activity of the Zr(IV) MOF drastically decreases due to the poisoning of the catalyst by the reaction by-products, which irreversibly adhere to the Zr(IV) ions by nullifying their catalytic activity. Thus, the need for a basic pH buffer for its operation greatly limits the time of use or useful life of the Zr(IV) MOF and makes it unattractive for large-scale manufacturing due to the technical difficulties associated with its use.

In an attempt to solve the need for a basic medium, MOFs treated with a basic agent such as Mg(OH)₂ incorporating OH⁻ anions in the MOF have been described. However, despite incorporating basic species these are consumed with the cycles of use, which leads again to the deactivation or loss of the catalytic activity of the MOF to degrade toxic compounds.

Thus, there is a need to provide a MOF useful for degrading toxic compounds that overcomes at least one of the drawbacks of the prior art.

In particular, the state of the art lacks a MOF capable of effectively degrading over time toxic compounds such as organophosphorus compounds. Nor has a MOF capable of effectively degrading over time toxic compounds under atmospheric conditions of pressure, temperature and relative humidity been proposed, nor a MOF capable of maintaining its catalytic activity indistinctly under acid or base extreme conditions for that purpose.

There is no MOF in the state of the art capable of effectively degrading in time organophosphorus compounds that is further suitable as an additive in a support material, whether the nature of the support material is solid, liquid or emulsifying.

Thus, there remains a need to provide a MOF capable of effectively degrading toxic compounds toxic such as, for example, organophosphorus compounds, with catalytic activity or extended shelf-life.

### Brief description of the invention

The MOF described in the present invention resolves at least one of the aforementioned drawbacks, offering other advantages which will be described below.

In a first aspect, the present invention provides a MOF solid which is characterized in that it includes in the metal-organic network or reticular structure titanium (IV) atoms and iron (III) atoms. The reticular structure comprises an organic part composed of a tricarboxylic ligand and an inorganic part composed of titanium atoms with valence +4 and iron atoms with valence +3 that both act as active centers of the MOF.

The MOF solid of the invention has the general formula (1):

[Ti^{IV}Fe^{III}₂(O)(L)₂(X)₃]S (1)

wherein:
X is each the same or different selected from:
O²⁻, OH⁻, H₂O, F⁻, Cl⁻, Br, I⁻, NO₃⁻, ClO₄⁻, BF₄⁻, SCN⁻, OH⁻, CH₃COO⁻, C₅H₇O₂⁻, SO₄²⁻and CO₃²⁻;
L is a tricarboxylic ligand; and
S is at least one molecule of a polar solvent selected from the group consisting of N,N'-dimethylformamide, N,N'-diethylformamide, N,N'-dimethylacetamide, N-methyl-2-pyrrolidone, methanol, ethanol, isopropanol, n-propanol, water or mixtures thereof.

In one embodiment, the MOF solid is crystalline.

With the MOF solid defined in the first aspect of the invention, the activation limitations in basic medium and/or the limitations of loss of catalytic activity over time during use are solved.

In a second aspect, the invention provides a process for obtaining the MOF solid of general formula (1) [Ti^{IV}Fe^{III}₂(O)(L)₂(X)₃]S, defined in the first aspect of the invention, characterized by the fact that it comprises the following steps:
*(i)*selecting a polar solvent, S, and optionally dissolving an inorganic acid in the polar solvent,
*(ii)* adding to the solvent or solution prepared in step *(i)*:
   a tricarboxylic ligand L,
   a Ti(IV) precursor,
   an Fe(II) precursor, anhydrous or hydrated, of formula FeX₂ or FeY,
      wherein:
      X is selected from among F⁻, Cl⁻, Br, I⁻, NO₃⁻, ClO₄⁻, BF₄⁻, SCN⁻, OH⁻, CH₃COO⁻and C₅H₇O₂⁻,
      Y is selected from among SO₄²⁻ and CO₃²⁻,
      and, next,
*(iii)* heating the solution to a temperature of 25°C to 150°C to give the MOF solid of general formula (1),

Wherein steps (i), (ii) and (iii) are carried out under anaerobic conditions,
wherein the tricarboxylic ligand is present in stoichiometric excess with respect to the Fe(II) precursor, and
wherein the inorganic acid, when present, is in a molar ratio with respect to the Fe(II) precursor comprised between 5 and 1500 mol/mol equivalents.

Anaerobic conditions are here understood as conditions in the absence of oxygen.

In a preferred embodiment, in step (i) a solution of a polar solvent, S, is prepared with an inorganic acid; the remaining steps are followed in the same manner in the process. Advantageously, the use of an inorganic acid under the conditions of the procedure provides the crystalline MOF solid.

In a third aspect, the invention relates to the use of the MOF solid of titanium (IV) and iron (III) defined in the first aspect of the invention as a catalyst for degrading toxic compounds.

In a fourth aspect, the invention relates to the use of the MOF solid of titanium (IV) and iron (III) defined in the first aspect of the invention as an additive with detoxifying properties of toxic compounds.

The MOF solid of titanium (IV) and iron (III) defined in the first aspect of the invention has catalytic properties in different support media whether it is of a solid, liquid or emulsifying nature, so that it is useful for use as an additive in plastics, paints or textiles.

The present invention also relates to a plastic, paint or textile additive with the MOF solid of titanium (IV) and iron (III) defined in the first aspect of the invention with detoxifying properties of toxic compounds.

In one embodiment, the toxic compound is an organophosphorus compound.

Throughout the disclosure, the MOF solid of titanium (IV) and iron (III) of the invention has also been referred to as MUV-17 (TiFe₂). The authors of the present invention have found that titanium (IV) atoms and iron (III) atoms are assembled in the MOF solid in a 1:2 ratio.

### Definitions

In the context of the present invention, the term "homogeneous distribution at the atomic level" is applied to the crystalline MOF solid and it is understood that the atomic ratio between titanium (IV) atoms and iron (III) atoms contained in the crystalline MOF solid is the same regardless of the zone of the crystalline MOF solid examined. When the MOF solid is amorphous the structure is not completely ordered and therefore it is not regular. In the amorphous MOF porosity is reduced, which limits the accessibility to the active centers of the MOF and reduces the catalytic activity of the MOF.

In the context of the present invention, "titanium (IV) and iron (III) MOF crystalline solid" means a structured metal-organic material solid with homogeneous distribution at the atomic level.

In the context of the present invention, "polar solvent" or "S" means any number of the same or different solvent molecules.

By "toxic compound" is meant an organic compound that is toxic to humans. In the present invention, the term "toxic" is understood in a broad sense without specifying whether the compound is toxic from a given concentration and is therefore understood to be a toxic compound. Toxic compounds susceptible to degradation with the MOF solid of the present invention include insecticides, pesticides such as ethoprophos or fenamiphos, chemical warfare agents based on phosphonate groups and their analogues such as diethylsulfide (DES, Mustard gas model), diisopropylfluorophosphate (DIFP, Sarin gas model), dimethyl-4-nitrophenyl phosphate (DMNP), dimethyl-methyl-phosphonate (DMMP), 2-chloroethyl-ethyl-sulfide (CEES), and derivatives thereof. These compounds are generally characterized by having a stereogenic center P(V) with a P=O bond and an alkyl (-R) and/or halogen (-X) and two alkoxy groups (-OR). In all cases, their degradation involves the substitution of the P-X or P-OR bonds by a P-OH bond to form the corresponding non-toxic phosphates or alkylphosphonic acids.

In the present invention, the term "degradation" or "detoxification" means the conversion of a toxic compound to a non-toxic compound. This conversion can take place via the following reaction: 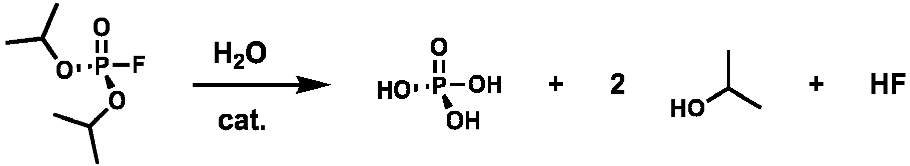 wherein H₂O is the water present in the ambient humidity or the water of an aqueous medium and the catalyst (cat.) is the MOF solid of the invention.

### Brief description of the drawings

In order to better understand the description made, a set of drawings has been provided which, schematically and solely by way of non-limiting example, represent a practical case of embodiment.

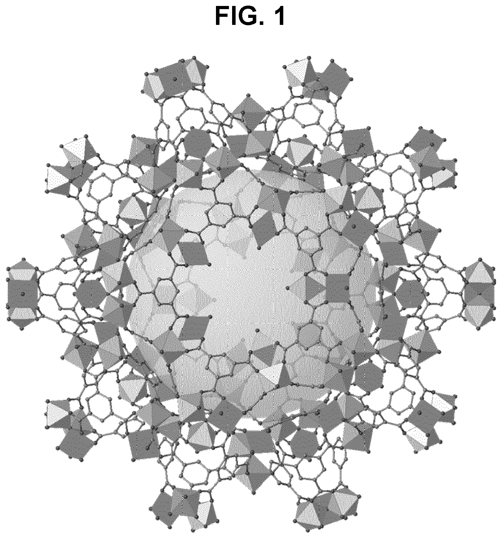

**Figure 1** is an image showing the three-dimensional structure of the crystalline and porous MOF solid of titanium (IV) and iron (III), MUV-17 (TiFe₂), obtained in Example 1 of the invention.

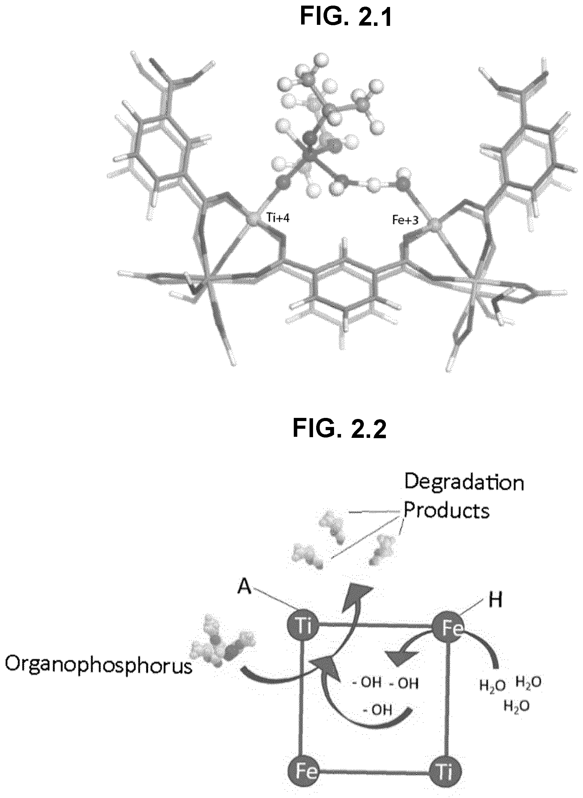

**Figure 2****.****1** is an image showing the active centers Ti⁺⁴ and Fe⁺³ in a part of the three-dimensional structure of the MOF crystalline solid MUV-17 (TiFe ₂), responsible for the degradation reaction by the dual catalyst function possessed by the MOF of the invention.
**Figure 2.2** is a schematic representation of the degradation reaction of a toxic compound by the dual catalyst function of the MOF crystalline solid MUV-17 (TiFe₂). In the degradation reaction the active center Ti⁺⁴ (A) activates the organophosphorus compound which is then hydrolyzed by the active center Fe⁺³ (H) where OH⁻ radicals are generated in the presence of H₂O which degrade the organophosphorus compound to non-toxic reaction by-products.

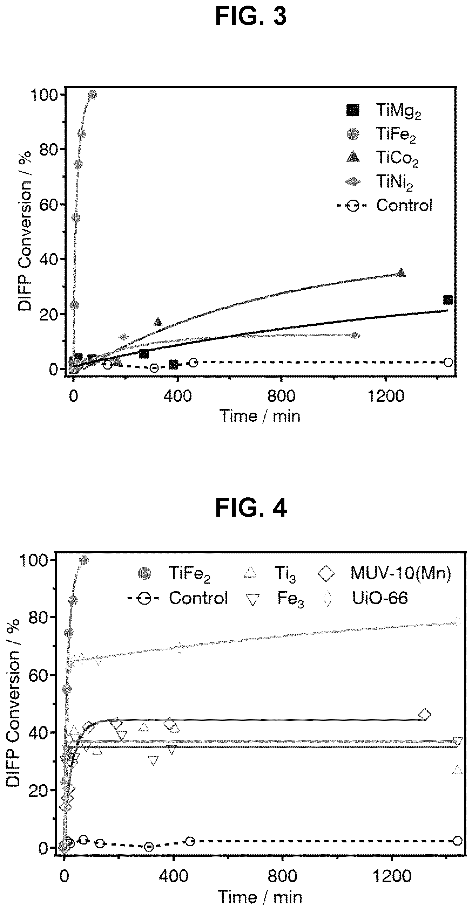

**Figure 3** is a graph showing the superior catalytic activity of the MOF crystalline solid, MUV-17 (TiFe ₂), obtained in Example 1 of the invention to degrade the organophosphorus compound DIFP (diisopropylfluorophosphate, Sarin gas model) in aqueous medium, expressed as a percentage of conversion to non-toxic reaction by-products, compared to the catalytic activity of the MOFs of TiMg ₂, TiNi₂ and TiCo₂, expressed in minutes.
**Figure 4** is a graph showing the superior catalytic activity of the MOF crystalline solid obtained in Example 1 MUV-17 (TiFe ₂) to degrade the organophosphorus compound DIFP (diisopropylfluorophosphate, Sarin gas model) in aqueous medium, expressed as a percentage of conversion to non-toxic reaction by-products, compared to the catalytic activity of its homometalic homologs: MIL-100 (Ti) orTi₃, MIL-100 (Fe) or Fe₃, and that of the MOF of Zr(IV) or UiO-66, and MOF MUV-10 (TiMn), expressed in minutes.

### Detailed description of the invention

The invention will now be described in more detail.

The MOF solid of the invention has the general formula (1):

[Ti^{IV}Fe^{III}₂(O)(L)₂(X)₃]S (1)

wherein:
X is each the same or different selected from:
O²⁻, OH⁻, H₂O, F⁻, Cl⁻, Br, I⁻, NO₃⁻, ClO₄⁻, BF₄⁻, SCN⁻, OH⁻, CH₃COO⁻, C₅H₇O₂⁻, SO₄²⁻and CO₃²⁻;
L is a tricarboxylic ligand; and
S is at least one molecule of a polar solvent selected from the group consisting of N,N'-dimethylformamide, N,N'-diethylformamide, N,N'-dimethylacetamide, N-methyl-2-pyrrolidone, methanol, ethanol, isopropanol, n-propanol, water or mixtures thereof.

In one embodiment, X is 2 OH⁻ and 1 H₂O, and L is a tricarboxylic ligand, the MOF solid having formula (1A): [Ti^{IV}Fe^{III}₂(O)(L)₂(OH)₂(H₂O)]S.

In another embodiment, X is 1 O²⁻ and 2 H₂O, and L is a tricarboxylic ligand, the MOF solid having the formula (1B): [Ti^{IV}Fe^{III}₂(O)(L)₂(O)(H₂O)₂]S.

"S" has the same meaning as defined in the general formula (1) above.

The different specific formulas that the MOF solid of the invention can have result from a different distribution of the terminal end of the ligand in the metal centers of the cluster in the

MOF reticular structure.

The tricarboxylic ligand L can be selected from a tricarboxylic C₆-aryl acid, a tricarboxylic C₃N₃-aryl acid or a derivative thereof of the type of tricarboxylic (C'₆-aryl)₃-C₆-aryl acid or tricarboxylic (C'₆-aryl)₃-C₃N₃-aryl acid.

The preferred tricarboxylic ligand L has one of the following structures:
tricarboxylic C₆-aryl:

| | |
|---|---|
| 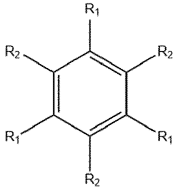 | **R₁** = -COOH |
| | **R₂** = -H, -(CH₂)₀₋₅-CH₃, -NH₂, -OH, -NO₂, -COOH, or halogen |

tricarboxylic C₃N₃-aryl:

| | |
|---|---|
| 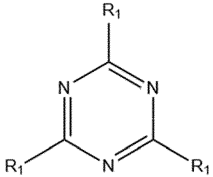 | **R₁** = -COOH |

tricarboxylic (C'₆-aryl)₃-C₆-aryl: 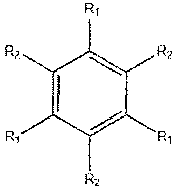 wherein **R₁** is selected from: 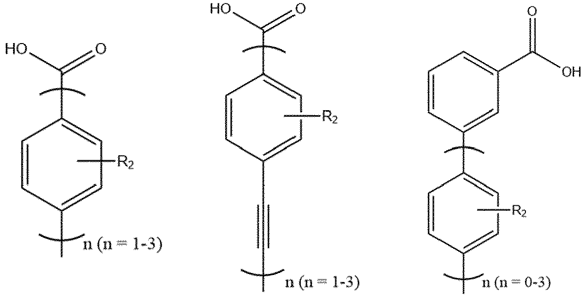 and **R₂** is -H, -(CH₂)₀₋₅-CH₃, -NH₂, -OH, -NO₂, -COOH or halogen
tricarboxylic (C'₆-aryl)₃-C₃N₃-aryl: 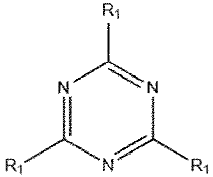 wherein **R₁** is selected from: 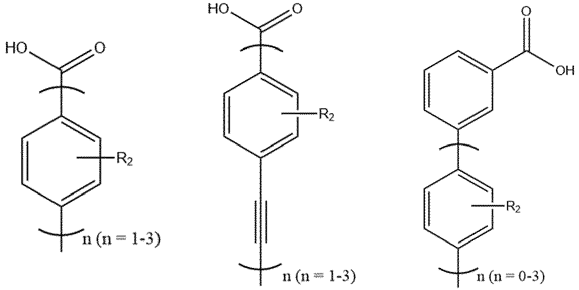 and **R₂** is -H, -(CH₂)₀₋₅-CH₃, -NH₂, -OH, -NO₂, -COOH or halogen

Preferably, the tricarboxylic ligand L is 1,3,5-benzenetricarboxylic acid, also called trimesic acid or trimesate ligand.

The MOF solid MUV-17 (TiFe₂) is porous.

The porosity of MOF MUV-17 (TiFe₂) is mainly determined by the tricarboxylic ligand employed. The MOF solid MUV-17 (TiFe₂) may have average pore sizes of 1 to 6 nm in diameter.

In one embodiment, the titanium (IV) and iron (III) clusters are connected by trimestate ligands that form a three-dimensional porous network with two average pore sizes of 2.4 and 2.9 nm in diameter.

The polar solvent "S" may be selected from the group comprising *N,N'*-dimethylformamide (DMF), N,N' -diethylformamide, *N,N'*-dimethylacetamide, N- methyl-2-pyrrolidone (NMP), methanol, ethanol, isopropanol, n-propanol, water and/or mixtures thereof such as, for example, a mixture of *N,N'*-dimethylformamide (DMF) and *N*-methyl-2-pyrrolidone (NMP).

Preferably, the polar solvent "S" is one or several N,N'-dimethylformamide (DMF) molecules or a mixture of *N,N'*-dimethylformamide (DMF) and *N*-methyl-2-pyrrolidone (NMP) molecules, preferably in a ratio of 1:1.

The MOF solid MUV-17 (TiFe₂) of the invention can include solvent molecules trapped in the reticular structure either due to the ambient humidity or as a residue of the solvent employed in the method for obtaining the MOF.

Preferably, the MOF solid of formulae (1), (1A) and (1B) is crystalline.

The crystalline nature provides a homogeneous distribution at the atomic level of the active Ti(IV) centers and the active Fe(III) centers in the MOF. In this homogeneous distribution, the separation between Ti(IV) and Fe(III) atoms is less than 2 nm. Both active centers are close enough to each other for there to be a simultaneous collaboration of both, providing a dual catalyst function throughout the structural network to the MOF for the degradation of toxic compounds. If the MOF solid is amorphous, the catalytic function is less and not the same throughout the metal-organic network of the MOF.

Surprisingly, the combination of titanium (IV) and iron (III) in the structure of the MOF results in a synergistic effect of dual catalyst, which confers on the MOF high catalytic activity and/or degradation capacity of toxic compounds and prolongs its useful life.

The synergistic effect of dual catalyst allows eliminating the use of basic co-catalysts or additives, the main problem of the materials used in the state-of-the-art to degrade toxic agents.

Catalytically, the active center of Ti⁺⁴ acts as Lewis acid, activating the organophosphorus compound and, therefore, making it susceptible to hydrolysis, and the active center of Fe⁺³ acts simultaneously with that of titanium as a Bronsted base, generating OH⁻ species which degrade the activated organophosphorus compound.

High catalytic activity indicates increased conversion of toxic compound to non-toxic reaction by-products. Unexpectedly, complete degradation can be obtained in a short period of time with the MOF crystalline solid of the invention (figures 3 and 4). The conversion of the toxic compounds to non-toxic reaction by-products is superior in the MOF crystalline solid. This conversion can be in the order of 80%, of 90%, or of 100%.

Therefore, the degree of the catalytic activity of the MOF defined in the first aspect is a function of the presence and proximity between the metal ions of Ti(IV) and Fe(III) and also of their homogeneous or heterogeneous distribution at the atomic level in the MOF.

Unexpectedly, the MOF solid of titanium (IV) and iron (III) exhibits low affinity for by-products resulting from degradation of the toxic compounds, which inhibits the catalyst poisoning process and makes it useful for use for several consecutive cycles without loss of catalytic activity, which if produced can be recovered by simple washing in distilled water (improved cyclability).

In its metal-organic structure the MOF crystalline solid has a stoichiometric ratio between Ti(IV) atoms and Fe(III) atoms of 1:2. The metal-organic structure contains combined both the activator, Ti^{IV}, and the hydrolyzing agent, Fe^{III}, a species capable of hydrolyzing water molecules at a distance close enough to act simultaneously. The presence of titanium (IV) in the structure makes it an activator due to its high state of oxidation. On the other hand, iron (III) is capable of generating hydroxyl groups (OH⁻) from water or moisture by an acid/base process. It is these hydroxyl groups that subsequently act as nucleophiles and break up the organophosphorus compound to result in non-toxic compounds.

The MOF crystalline solid of Ti(IV) and Fe(III) has the ability to hydrolyze phosphate ester bonds through cooperative catalysis between the two metal ions that make up the active centers of the MOF.

Advantageously, the ambient humidity is sufficient to spontaneously initiate the activation and hydrolysis reactions in the MOF of the invention, MUV-17 (TiFe ₂), which lead to the degradation of the toxic compounds. The existence of the two functions necessary to degrade organophosphorus compounds in the structure of MOF MUV-17 (TiFe₂) confers superior catalytic stability.

Advantageously, the MOF crystalline solid of titanium (IV) and iron (III) exhibits catalytic stability under acidic and basic extreme conditions.

In a second aspect, the invention provides a process for obtaining the MOF solid of titanium (IV) and iron (III) defined in the first aspect of the invention which is characterized in that it comprises the following steps:
(i) selecting a polar solvent, S, and optionally dissolving an inorganic acid in the polar solvent,
*(ii)* adding to the solvent or solution prepared in step *(i)*:
   a tricarboxylic ligand L,
   a Ti(IV) precursor,
   an Fe(II) precursor, anhydrous or hydrated, of formula FeX₂ or FeY,
      wherein:
      X is selected from among F⁻, Cl⁻, Br, I⁻, NO₃⁻, ClO₄⁻, BF₄⁻, SCN⁻, OH⁻, CH₃COO⁻and C₅H₇O₂⁻,
      Y is selected from among SO₄²⁻ and CO₃²⁻,
      and, then,
*(iii)* heating the solution to a temperature of 25°C to 150°C to give the MOF solid of general formula (1),

wherein steps (i), (ii) and (iii) are carried out under anaerobic conditions,
wherein the tricarboxylic ligand is present in stoichiometric excess with respect to the Fe(II) precursor, and
wherein the inorganic acid, when present, is in a molar ratio with respect to the Fe (II) precursor comprised between 5 and 1500 mol equivalents.

The method of production is based on a one-pot *synthesis*, by direct reaction.

Manufacturing MOFs incorporating both metals in Ti⁺⁴ and Fe⁺³ oxidation states is very complex synthetically due to the high acidity of both metals. In fact, the authors of the present invention have been able to verify that the combination of simple salts of both metals in oxidation states Ti⁺⁴ and Fe⁺³ did not give good results.

To overcome this problem, the authors of the present invention have found that the combination of a Ti⁺⁴ precursor with a Fe⁺² precursor, of lower acidity than that of Fe⁺³, in anaerobic atmosphere before heating the solution containing them facilitates the crystallization of the three-dimensional structure with the TiFe₂ metal cluster and, subsequently, when exposed to the oxygen present in the air, the Fe(II) is oxidized to Fe(III). The authors of the present invention have observed that if the synthesis of the MOF is not performed under anaerobic conditions, i.e. without bubbling the solution with argon, helium or nitrogen or any inert gas that ensures the synthesis in anoxia, then the segregated formation of iron and titanium in the MOF occurs, i.e. a homogeneous distribution at the atomic level of both metal ions in the reticular structure of the MOF is not obtained. Thus, the invention solves the additional problem of the segregated formation of Ti and Fe in a MOF and makes it possible to obtain with a simple method an MOF solid whose structure combines Ti(IV) and Fe(III) metal ions with homogeneous distribution at the atomic level.

Advantageously, the use of anaerobic conditions allows the iron ions in the oxidation state Fe²⁺ to form as active centers in the MOF, without this interfering in the simultaneous formation of active centers of titanium in the oxidation state Ti⁴⁺ in the MOF. At the end of the synthesis and when the MOF solid comes into contact with the air of the environment, an MOF is obtained with Fe³⁺ and Ti⁴⁺ ions as active centers of the MOF.

In one embodiment, steps (i) and (ii) are performed simultaneously. The inorganic acid, when present, is mixed with the polar solvent, the tricarboxylic ligand L, the Ti(IV) precursor and the Fe(II) precursor and then the solution is heated following step (iii). Throughout the synthesis, the solution is bubbled with argon, helium or nitrogen in order to ensure anaerobic conditions.

The inorganic acid has the function of modulating the crystalline growth of MOF.

The inorganic acid can be selected from hydrochloric acid, formic acid, acetic acid, propanoic acid, benzoic acid and derivatives thereof.

The Ti(IV) precursor can be selected from a Ti(IV) organometallic precursor such as a Ti(IV) alkoxide, preferably, Ti(IV) isopropoxide, Ti(IV) methoxide, Ti(IV) ethoxide, Ti(IV) n-propoxide, Ti(IV) n-butoxide, Ti(IV)-triethanolaminate isopropoxide, Ti(IV) tert-butoxide, Ti(IV) oxo-diacetylacetonate; the Ti(IV) oxo-di-acethylacetonate precursor can also be a titanium compound such as Ti(IV) oxo di-acethylacetonate tetrachloride, bis(cyclopentadienyl)-Ti(IV) dichloride, cyclopentadienyl-Ti(IV) trichloride or Ti(IV) oxosulphate or similar; or a Ti(IV) polynuclear compound stable in air such as a Ti(IV) hexanuclear complex.

The Fe²⁺ precursor may be selected from a compound of formula FeX₂ or FeY, where X = F⁻, Cl⁻, Br, I⁻, NO₃⁻, ClO⁴⁻, BF₄⁻, SCN⁻, OH⁻, CH₃COO⁻ or C₅H₇O²⁻ and Y = SO₄²⁻ or CO₃²⁻. The Fe(ll) precursor of formula FeX₂ or FeY may be in anhydrous or hydrated form.

In one embodiment, the Fe(II) precursor is an iron (II) halide such as iron (II) fluoride, iron (II) chloride, iron (II) bromide, or iron (II) iodide.

In one embodiment, the Ti(IV) precursor and the Fe(II) precursor are added in a ratio of 1_{Ti}:99_{Fe} to 50_{Ti}:50_{Fe}, wherein said ratio indicates moles of Ti(IV) in the titanium (IV) precursor to moles of Fe(II) in the iron (II) precursor.

In one embodiment, the stoichiometric ratio between the Fe(II) precursor and the ligand is comprised between 1:1.1 - 1:6, more preferably between 1:2 - 1:3.

In another aspect, the invention is directed to the use of the MOF solid defined in the first aspect in heterogeneous catalysis.

In another aspect, the invention relates to the use of the MOF solid defined in the first aspect for the degradation of toxic compounds.

In another aspect, the invention relates to the use of the MOF solid defined in the first aspect as an additive with catalytic activity for degrading toxic compounds.

In another aspect, the invention relates to the use of the MOF solid defined in the first aspect as an additive in a plastic material, paints and/or a textile material with detoxification properties of toxic compounds.

In another aspect, the invention relates to a plastic material comprising an amount of the MOF solid defined in the first aspect as an additive with detoxification properties of toxic compounds.

In another aspect, the invention relates to a paint comprising an amount of the MOF solid defined in the first aspect as an additive with detoxification properties of toxic compounds.

In another aspect, the invention relates to a textile material comprising an amount of the MOF solid defined in the first aspect as an additive with detoxification properties of toxic compounds.

Preferably, the toxic compound is an organophosphorus compound.

The MOF solid of Ti(IV) and Fe(III) according to the first aspect of the invention can also be used in water treatment, in the elimination of cross-contamination in equipment, as an additive in fibers for defensive tissues or as a material for filters and masks.

The MOF solid of Ti(IV) and Fe(III), MUV-17 (TiFe₂), according to the first aspect of the present invention hasat least one of the following features:
- Crystalline solid comprising Ti(IV) and Fe(III) atoms homogeneously distributed at the atomic level throughout the metal-organic network of the MOF;
- Crystalline solid having a stoichiometric ratio of Ti(IV) atoms to Fe(III) atoms of 1:2.
- High catalytic activity and cyclability in catalytic/degradation processes;
- Conversion of toxic compounds into non-toxic compounds exceeding 80%;
- Degradation of toxic compounds by more than 90%, preferably 100%;
- Longer service life in use, extended service life in time;
- Stable catalytic activity in the presence of moisture, in aqueous medium, and in an acidic or base aggressive medium with pHs close to 1 and 13 respectively;
- It does not require a basic buffered medium for catalysis/degradation;
- Useful as an additive, especially as an additive in plastics, paints or textiles;
- Catalytic stability in mediums other than a solution, such as an emulsion, solid or liquid;
- Thermal stability between -50 and 500°C.
- Porosity with a BET surface area greater than or equal to 3,000 m²/g;
- Porous with average pore size ranging from 1 to 6 nm in diameter.
- Crystalline solid with average particle size between 0.1-500 µm.

Preferred embodiments of the present invention are disclosed below.

### Examples

### Example 1

### Preparation of the MOF crystalline solid of Ti(IV) and Fe(III) - MUV-17 (TiFe₂)

125 mg of trimesic acid (H₃L), 48 mg of iron (II) chloride tetrahydrate (FeCl₂·4H₂O) and 36 µL of titanium (IV) isopropoxide (Ti(OⁱPr)₄) was dissolved in a mixture of *N,N* -dimethyl-formamide (12 mL) and acetic acid (7 mL, 1000 mol per mole of titanium) previously bubbled with argon for several minutes. The reaction mixture was introduced into an oven at 120 °C and kept at that temperature for 48 hours. The obtained product was washed 2 to 3 times with N,N-dimethylformamide and then purified by a solid-liquid extraction by the soxhlet method for 4-5 hours with hot methanol. Finally, the product was allowed to dry at room temperature. The MOF catalyst MUV-17 obtained (TiFe₂) showed the following stoichiometric formula: [TiFe₂(O)(L)₂(OH)₂(H₂O)]S, where S refers to the presence of molecules of the solvent used in the procedure and L tricarboxylic ligand.

### Catalytic degradation tests

Catalytic activity assays were carried out to determine the ability of the catalyst MOF prepared in Example 1 in the degradation of organophosphorus compounds. Nerve agents were tested through simulants of these organophosphorus compounds among which are DIFP = diisopropyl-fluorophosphate, DMNP = dimethyl-4-nitrophenyl phosphate, DMMP = dimethyl-methyl-phosphonate, CEES = 2-chloroethyl-ethyl-sulfide, and derivatives thereof.

Suspensions of the MOF MUV-17 (TiFe ₂) prepared in Example 1, and other prior art MOFs were prepared: MUV-102(TiMg), MUV-102(TiNi) and MUV-102 (TiCo) of formulae [Ti^{IV}Mg^{II}₂(O)(L)₂(H₂O)₃], [Ti^{IV}Ni^{II}₂(O)(L)₂(H₂O)₃] and [Ti^{IV}Co^{II}₂(O)(L)₂(H₂O)₃], respectively, where L = trimesic acid, in water. Subsequently, a known amount of the nerve agent simulant was added with a 1:1 molar ratio (MOF:Simulant). Activation and hydrolysis reactions of the nerve agent simulant were carried out at room pressure and temperature.

The catalytic activity of the catalyst was monitored by gas chromatography measuring the decrease in nerve agent concentration at regular time intervals and represented graphically **(****figure 3**). Figure 3 shows the high degradation capacity of the MUV-17 catalyst (TiFe ₂), represented by a solid circle and TiFe₂ reading, compared to that of the prior art MOFs: TiMg₂, TiNi₂ and TiCo₂ and without catalyst (control). The results depicted in figure 3 reveal that combinations of Ti⁺⁴ with divalent metals (Mg²⁺, Co²⁺ and Ni²⁺) exhibit very low reactivity to organophosphorus compounds compared to the activity of the MUV-17 MOF (TiFe₂) of the invention.

A second degradation catalytic assay was carried out. In this case, the catalytic activity of the MUV-17 catalyst (TiFe ₂) of the invention was compared with the catalytic activity of its homometallic counterparts: MIL-100 (Ti) or Ti₃, of formula [Ti^{IV}(O)(L)₂(O)(OH)₂], L = trimesic acid, MIL-100 (Fe) or Fe₃, of formula [Fe^{III}(O)(L)₂(O)(OH)(H₂O)₂], L = trimesic acid, and also with respect to the MOF of Zr(IV), UiO-66, of formula [Zr^{IV}₆(O)₄(OH)₄(L)₆], L = terephthalic acid and the heterometallic MOF of Ti and Mn", MUV-10 (TiMn), of formula [Ti₃Mn₃II(O)₃(L)₄(H₂O)₃], L = trimesic acid and without catalyst (control). The data obtained were represented graphically (**figure 4**). Figure 4 shows that the MUV-17 catalyst (TiFe ₂) of the invention, represented by a solid circle and TiFe₂ reading, allows a 100% conversion of the organophosphorus compound and, therefore, a complete degradation thereof. In addition, Figure 4 also shows the surprising improvement of the MUV-17 catalyst (TiFe₂) of the invention, represented by a solid circle and TiFe₂ reading, compared to the reference material employed in the state of the art, a MOF of Zr(IV) known by UiO-66, which is poisoned and reaches only a conversion of the order of 65%.

The MOF crystalline solid of Ti(IV) and Fe(III) shows catalytic activity effective over time to degrade organophosphorus compounds. The MOF crystalline solid of Ti(IV) and Fe(III) is able to regenerate with just one wash in distilled water thus improving its cyclability and prolonging its useful life. These characteristics make it particularly useful for degrading large-scale organophosphorus compounds.

Although reference has been made to a specific embodiment of the invention, it is evident to a person skilled in the art that the optional characteristics are susceptible to numerous variations and modifications, and that all the aforementioned details can be replaced by other technically equivalent details, without departing from the scope of protection defined by the appended claims.

## Claims

1. A titanium (IV) and iron (III) MOF solid of general formula (1):
[Ti^{IV}Fe^{III}₂(O)(L)₂(X)₃]S (1)
wherein:
X is each the same or different selected from:
O²⁻, OH⁻, H₂O, F-, Cl⁻, Br, I⁻, NO₃⁻, ClO₄⁻, BF₄⁻, SCN⁻, OH⁻, CH₃COO⁻, C₅H₇O₂⁻, SO₄²⁻and CO₃²⁻;
L is a tricarboxylic ligand; and
S is at least one molecule of a polar solvent selected from the group consisting of N,N'-dimethylformamide, N,N'-diethylformamide, N,N'-dimethylacetamide, N-methyl-2-pyrrolidone, methanol, ethanol, isopropanol, n-propanol, water or mixtures thereof.

2. MOF solid according to claim 1, wherein X is 2 OH⁻ and 1 H₂O, and L is a tricarboxylic ligand, the MOF solid having formula (1A): [Ti^{IV}Fe^{III}₂(O)(L)₂(OH)₂(H₂O)]S.

3. MOF solid according to claim 1, wherein X is 1 O²⁻ and 2 H₂O, and L is a tricarboxylic ligand, the MOF solid having formula (1B): [Ti^{IV}Fe^{III}₂(O)(L)₂(O)(H₂O)₂]S.

4. MOF solid according to any one of claims 1 to 3, wherein the MOF solid is crystalline and the titanium (IV) and iron (III) are homogeneously distributed at the atomic level in the MOF.

5. MOF solid according to any one of the preceding claims, wherein the tricarboxylic ligand L is selected from a tricarboxylic C₆-aryl acid, a tricarboxylic C₃N₃-aryl acid or a derivative thereof of the type of tricarboxylic (C'₆-aryl)₃-C₆-aryl acid or tricarboxylic (C'₆-aryl)₃-C₃N₃-aryl acid.

6. MOF solid according to any one of the preceding claims, wherein the tricarboxylic ligand L has one of the following structures:
tricarboxylic C₆-aryl:
| | |
|---|---|
| 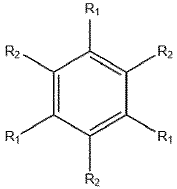 | **R₁** = -COOH |
| | **R₂** = -H, -(CH₂)₀₋₅-CH₃, -NH₂, -OH, -NO₂, -COOH, or halogen |
tricarboxylic C₃N₃-aryl:
| | |
|---|---|
| 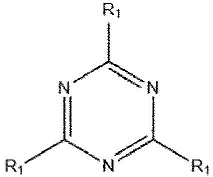 | **R₁** = -COOH |
tricarboxylic (C'₆-aryl)₃-C₆-aryl: 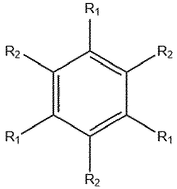 wherein **R₁** is selected from: 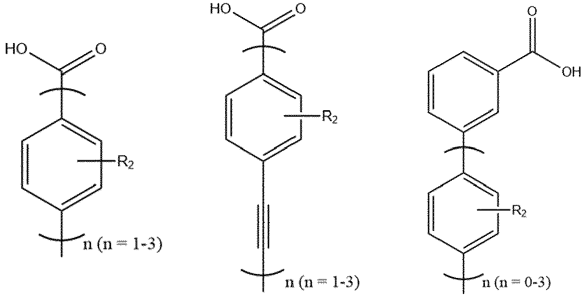 and **R₂** is -H, -(CH₂)₀₋₅-CH₃, -NH₂, -OH, -NO₂, -COOH or halogen
tricarboxylic (C'₆-aryl)₃-C₃N₃-aryl: 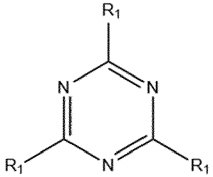 wherein **R₁** is selected from: 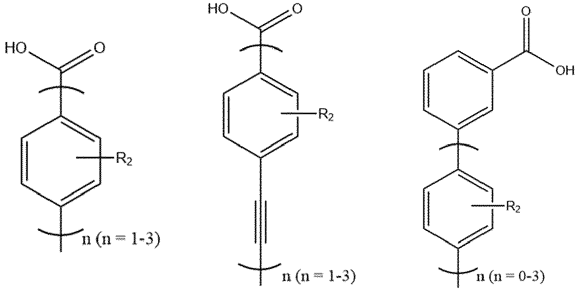 and **R₂** is -H, -(CH₂)₀₋₅-CH₃, -NH₂, -OH, -NO₂, -COOH or halogen

7. MOF solid according to any one of the preceding claims, wherein the tricarboxylic ligand L is 1,3,5-benzene-tricarboxylic acid or trimestate.

8. MOF solid according to any one of the preceding claims, with catalytic activity for degrading toxic compounds in the presence of moisture and/or in aqueous medium.

9. MOF solid according to claim 8 with catalytic activity for activating and hydrolyzing toxic compounds and conversion of toxic compound to non-toxic compound greater than 80%.

10. Process for obtaining an MOF solid of titanium (IV) and iron (III) of general formula (1), [Ti^{IV}Fe^{III}₂(O)(L)₂(X)₃]S, defined in any one of claims 1 to 9, comprising the steps of:
(i) selecting a polar solvent, S, and optionally dissolving an inorganic acid in the polar solvent,
*(ii)* adding to the solvent or solution prepared in step *(i)*:
a tricarboxylic ligand L,
a Ti(IV) precursor,
an Fe(II) precursor, anhydrous or hydrated, of formula FeX₂ or FeY,
wherein:
X is selected from F-, Cl⁻, Br, I⁻, NO₃⁻, ClO₄⁻, BF₄⁻, SCN⁻, OH⁻, CH₃COO⁻ and
C₅H₇O₂⁻,
Y is selected from SO₄²⁻ and CO₃²⁻,
and, then,
*(iii)* heating the solution to a temperature of 25°C to 150°C to give the MOF solid of general formula (1),
wherein steps (i), (ii) and (iii) are carried out under anaerobic conditions,
wherein the tricarboxylic ligand is present in stoichiometric excess with respect to the Fe(II) precursor, and
wherein the inorganic acid, when present, is in a molar ratio with respect to the Fe(II) precursor comprised between 5 and 1500 mol/mol equivalents.

11. Process according to claim 10, wherein steps (i), (ii) and (iii) are carried out under anaerobic conditions by treating the solution with argon, helium or nitrogen.

12. Process according to any one of claims 10-11, wherein steps (i) and (ii) are performed simultaneously so that inorganic acid, polar solvent, tricarboxylic ligand L, Ti(IV) precursor and Fe(II) precursor are mixed and then step (iii) of heating is continued.

13. Process according to any one of claims 10 to 12, wherein the inorganic acid is selected from hydrochloric acid, formic acid, acetic acid, propanoic acid, benzoic acid and derivatives thereof.

14. Process according to any one of claims 10 to 13, wherein the Ti(IV) precursor is selected from an organometallic precursor of Ti(IV) such as a Ti(IV) alkoxide, a titanium (IV) compound such as Ti(IV) tetrachloride, bis(cyclopentadienyl)-Ti(IV) dichloride, cyclopentadienyl-Ti(IV) trichloride, or Ti(IV) oxosulfate or the like, or an air-stable Ti(IV) polynuclear compound such as a Ti(IV) hexanuclear complex.

15. Process according to any one of claims 10 to 14, wherein the polar solvent is selected from the group comprising *N,N'*-dimethylformamide (DMF), *N,N'* -diethylformamide, *N,N'-*dimethylacetamide, N- methyl-2-pyrrolidone (NMP), methanol, ethanol, isopropanol, n-propanol, water and/or mixtures thereof such as a mixture of *N,N'*-dimethylformamide (DMF) and *N*-methyl-2-pyrrolidone (NMP).

16. Process according to any one of claims 10 to 15, wherein the Ti(IV) precursor and the Fe(II) precursor are added in a ratio comprised between 1_{Ti}:99_{Fe} and 50_{Ti}:50_{Fe}, expressed in moles of Ti(IV) in the titanium (IV) precursor with respect to moles of Fe(II) in the iron (II) precursor.

17. Process according to any one of claims 10 to 16, wherein the stoichiometric ratio between the Fe(II) precursor and the tricarboxylic ligand is comprised between 1:1.1 - 1:6, preferably between 1:2 - 1:3.

18. Use of the MOF solid according to any one of claims 1 to 9 in heterogeneous catalysis.

19. Use of the MOF solid according to any one of claims 1 to 9 for the degradation of toxic compounds.

20. Use of the MOF solid according to any one of claims 1 to 9 as an additive with catalytic activity for degrading toxic compounds.

21. Use of the MOF solid according to claim 20 as an additive in a plastic material, paints and/or a textile material with detoxification properties of toxic compounds.

22. A plastic material including a MOF solid of titanium (IV) and iron (III) defined in claims 1 to 9 as an additive with detoxification properties of toxic compounds.

23. A paint including an MOF solid of titanium (IV) and iron (III) defined in claims 1 to 9, as an additive with detoxification properties of toxic compounds.

24. A textile material including a solid MOF of titanium (IV) and iron (III) defined in claims 1 to 9, as an additive with detoxification properties of toxic compounds.
